# DEMANDE DE BREVET EUROPEEN

(11) **EP 1 591 100 A1**
(43) Date de publication de la demande: **02.11.2005**
(21) Numéro de dépôt: 05290870.4
(22) Date de dépôt: 19.04.2005
(51) Int. Cl.: A61K 7/043

(54) **Composition de vernis à ongles comprenant un polymère ayant des unités LCST**

(30) Priorité: 27.04.2004 FR 0450800
(71) Demandeur: L'OREAL, 75008 Paris (FR)
(72) Inventeur: L'Alloret, Florence, 75013 Paris (FR)
(74) Mandataire: Boulard, Denis

(57) **Abrégé**

L'invention a pour objet une composition de vernis à ongles comprenant, dans un milieu aqueux cosmétiquement acceptable, un polymère hydrosoluble ou hydrodispersible comprenant des unités hydrosolubles ou hydrodispersibles et des unités à LCST.

L'invention a également pour objet un procédé de maquillage ou de soin des ongles.

## Description

La présente invention a pour objet un vernis à ongles comprenant un polymère hydrosoluble ou hydrodispersible comprenant des unités hydrosolubles ou hydrodispersibles et des unités à LCST. L'invention a également pour objet un procédé de maquillage ou de soin des ongles.

La composition peut être employée comme produit de maquillage des ongles tel qu'un vernis à ongles coloré, comme base pour les ongles ou "base-coat" en terminologie anglo-saxonne, comme composition de finition, encore appelée "top-coat", à appliquer sous ou sur le produit de maquillage des ongles ou bien encore comme produit de soin cosmétique des ongles tel qu'une base traitante destinée à protéger, à renforcer et/ou réparer les ongles.
Cette composition peut s'appliquer sur les ongles d'êtres humains ou bien encore sur des faux ongles.

Les compositions de vernis à ongles comprennent en général des particules solides telles que les pigments, les nacres, les charges, qui sont en dispersion dans le milieu continu aqueux ou le milieu solvant organique de la composition.
Or ces particules ont tendance à sédimenter au cours du temps, du fait de leur densité qui est supérieure à celle du milieu continu dans lequel elles sont dispersées. Cette sédimentation se traduit par une modification de l'aspect macroscopique de la composition, et en particulier, dans le cas de vernis à ongles colorés, par une hétérogénéité de la couleur du vernis. Ce phénomène est accentué lorsque la température augmente, en particulier lorsqu'elle dépasse 25°C.
Afin d'améliorer la stabilisation des compositions, le formulateur dispose d'agents épaississants ou gélifiants ; toutefois, l'incorporation de ces agents en une quantité nécessaire à la stabilisation du vernis entraîne généralement une augmentation de la viscosité, et donc une augmentation de la consistance du produit, qui peut se traduire par des difficultés d'application sur l'ongle pour l'utilisatrice.

C'est pourquoi on cherche à obtenir une composition de vernis à ongles qui présente une bonne stabilité et une bonne homogénéité de la couleur dans le temps, notamment à des températures supérieures à 25°C, ainsi qu'une consistance permettant à l'utilisatrice une bonne application du vernis sur les ongles.

Le demandeur a constaté de façon surprenante que l'utilisation d'un polymère hydrosoluble ou hydrodispersible particulier permet l'obtention d'une composition présentant une consistance satisfaisante dans le temps. En particulier, ce polymère, est tel que la viscosité de la composition qui le contient augmente lorsqu'elle est soumise à une température supérieure à la température de gélification dudit polymère, contrairement aux vernis à ongles en milieu aqueux de l'art antérieur, dont la viscosité diminue lorsqu'ils sont soumis à une augmentation de température.

C'est pourquoi un objet de la présente invention est une composition de vernis à ongles comprenant, dans un milieu aqueux cosmétiquement acceptable, un polymère hydrosoluble ou hydrodispersible, le polymère étant tel que la viscosité de ladite composition augmente lorsqu'elle est soumise à une température supérieure à la température de gélification dudit polymère.

En particulier, le polymère est tel que, lorsque la composition est soumise à une température supérieure ou égale à la température de gélification dudit polymère + 20°C, la viscosité de ladite composition augmente d'au moins 10% par rapport à sa viscosité mesurée à la température de gélification du polymère, pour une vitesse de cisaillement égale à 10 s⁻¹.

De préférence, le polymère comprend des unités hydrosolubles ou hydrodispersibles et des unités à LCST (Lower Critical Solution Température ou température inférieure critique de démixtion), lesdites unités à LCST présentant dans l'eau une température de démixtion (ou point de trouble) de 5 à 40°C à une concentration massique de 1 %.

L'invention a donc également pour objet une composition de vernis à ongles comprenant, dans un milieu aqueux cosmétiquement acceptable, un polymère hydrosoluble ou hydrodispersible comprenant des unités hydrosolubles ou hydrodispersibles et des unités à LCST, lesdites unités à LCST présentant dans l'eau une température de démixtion (ou point de trouble) de 5 à 40°C à une concentration massique de 1 %.

L'invention a aussi pour objet un procédé cosmétique de maquillage ou de soin non thérapeutique des ongles comprenant l'application sur les ongles d'au moins une couche de ladite composition.

L'invention a encore pour objet l'utilisation d'un polymère hydrosoluble ou hydrodispersible comprenant des unités hydrosolubles ou hydrodispersibles et des unités à LCST, lesdites unités à LCST présentant dans l'eau une température de démixtion de 5 à 40 °C à une concentration massique de 1 %, dans une composition de vernis à ongles comprenant un milieu aqueux cosmétiquement acceptable, pour conférer à ladite composition une homogénéité de couleur dans le temps, en particulier à des températures supérieures à 25°C.

Par "milieu aqueux cosmétiquement acceptable", on entend au sens de l'invention un milieu aqueux compatible avec les ongles.

Par « homogénéité en couleur », on entend une uniformité de la couleur de la composition depuis le bas jusqu'au haut du flacon la contenant, cette caractéristique étant évaluée par observation macroscopique à l'oeil nu.

La composition de vernis à ongles selon l'invention comprend un polymère hydrosoluble ou hydrodispersible comprenant des unités hydrosolubles ou hydrodispersibles et des unités à LCST, lesdites unités à LCST présentant dans l'eau une température de démixtion de 5 à 40°C à une concentration massique de 1 %.
Comme exposé ci-après, ces polymères possèdent en phase aqueuse des propriétés de gélification stimulées par une élévation de la température, ce qui leur permet d'assurer l'homogénéité en couleur des compositions de vernis à ongles, sans conduire à des formulations très consistantes et difficiles à appliquer à la température ambiante (25°C).

Des polymères hydrosolubles ou hydrodispersibles comportant des unités hydrosolubles ou hydrodispersibles et des unités à LCST ont déjà été décrits dans les documents suivants : D. HOURDET et al., Polymer, 1994, vol. 35, n°12, pages 2624 à 2630 [1] ; F. L'ALLORET et al., Coll. Polym. Sci., 1995, vol. 273, n°12, pages 1163-1173, [2] ; F. L'ALLORET et al., Revue de l'Institut Français du Pétrole, 1997, vol. 52, n°2, pages 117-128 [3] ; EP-A-0 583 814 [4] et EP-A-0 629 649 [5].
Ainsi, comme il est décrit dans ces documents, ces polymères hydrosolubles ou hydrodispersibles comportent des unités hydrosolubles ou hydrodispersibles et des unités à LCST qui présentent dans l'eau une température inférieure critique de démixtion. Ces unités à LCST sont des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère est appelée "LCST" ("Lower Critical Solution Temperature"). Pour chaque concentration en polymère, une température de démixtion par chauffage est observée ; elle est supérieure à la LCST qui est le point minimum de la courbe. En dessous de cette température, le polymère est soluble dans l'eau, au-dessus de cette température, le polymère perd sa solubilité dans l'eau.

Les températures de démixtion sont déterminées par spectroscopie UV visible en mesurant, à une longueur d'onde égale à 500 nm, l'absorbance de solutions aqueuses comprenant 1 % en poids desdites unités à LSCT. La température de démixtion est identifiée à la température à laquelle l'absorbance de la solution est égale à 2.

Selon l'invention, on utilise un polymère dont les unités à LCST présentent dans l'eau une température de démixtion de 5 à 40 °C à une concentration massique de 1 %,

Ainsi, ces polymères présentent des propriétés de gélification dans l'eau provoquées par une augmentation de la température. Ces propriétés sont observées au-delà de la température de démixtion des unités à LCST et sont dues à l'association des unités à LCST au sein de microdomaines hydrophobes, formant ainsi des noeuds de réticulation entre les chaînes principales. Ce pouvoir gélifiant est réversible en fonction de la température, en accord avec l'origine thermodynamique du processus de réticulation physique mis en jeu.

Ces polymères permettent d'obtenir des compositions de vernis à ongles dont la gamme de texture à la température ambiante (25°C) est aussi large que possible, fluide ou gélifiée de consistance plus ou moins grande, et conservant leur homogénéité en couleur au delà de la température ambiante. La texture de ces compositions à 25°C peut être ajustée à souhait par l'introduction d'un autre gélifiant tel que les polyuréthanes associatifs (Serad FX 1100 fourni par la société Servo) ou encore les argiles hydrophiles (Laponite XLS fournie par la société Rockwood).

Les propriétés gélifiantes des polymères hydrosolubles ou hydrodispersibles comportant des unités à LCST sont observées lorsque la concentration est suffisante pour permettre des interactions entre des unités à LCST portées par des macromolécules différentes. La concentration minimale nécessaire, appelée concentration critique d'agrégation ou CAC, est évaluée par des mesures de rhéologie : il s'agit de la concentration à partir de laquelle la viscosité d'une solution aqueuse des polymères devient supérieure à la viscosité d'une solution aqueuse du polymère équivalent ne comportant pas d'unités à LCST.

Au-delà de la CAC, les polymères utilisés dans le cadre de l'invention présentent des propriétés de gélification lorsque la température devient supérieure à une valeur critique appelée température de gélification ou T_{gel}. D'après les données de la littérature, un bon accord existe entre la température de gélification et la température de démixtion des unités à LCST, dans les mêmes conditions de concentrations. La température de gélification d'une solution aqueuse d'un polymère de l'invention est déterminée par des mesures de rhéologie : il s'agit de la température à partir de laquelle la viscosité de la solution du polymère devient supérieure à la viscosité d'une solution du polymère équivalent ne comportant pas d'unités à LCST.

Les températures de gélification sont déterminées de la façon suivante. On considère la température de gélification atteinte lorsque la différence entre la viscosité de la solution de polymère et la viscosité d'une solution du polymère équivalent ne comportant pas d'unités à LCST, est supérieure à 5%.
La viscosité de la composition est mesurée à l'aide d'un rhéomètre Haake RS150 muni d'une géométrie cône/plan 3,5 cm/2° ou 6 cm/2° et d'un système de contrôle de la température. Les mesures de viscosité sont réalisées dans le mode écoulement en imposant un gradient (ou vitesse) de cisaillement égal à 10⁻¹ s⁻¹.

Selon l'invention, on utilise, de préférence, des polymères présentant une température de gélification de 5 à 50°C, de préférence de 15 à 40°C, pour une concentration massique dans l'eau de 2%.

Les polymères utilisés dans le cadre de l'invention peuvent se présenter sous la forme de polymères séquencés (ou blocs) comprenant des unités hydrosolubles ou hydrodispersibles et des unités à LCST alternées ou sous la forme de polymères greffés constitués d'un squelette éventuellement réticulé formé d'unités hydrosolubles ou hydrodispersibles et porteur de greffons constitués d'unités à LCST ou vice-versa.

### a) Unités hydrosolubles ou hydrodispersibles

Les unités hydrosolubles ou hydrodispersibles de ces polymères, selon l'invention, sont de préférence des unités solubles dans l'eau, de 5°C à 80°C, à raison d'au moins 10 g/L, de préférence d'au moins 20 g/L.
Toutefois, on peut également employer comme unités hydrosolubles ou hydrodispersibles des unités ne possédant pas obligatoirement la solubilité mentionnée ci-dessus, mais qui en solution aqueuse à 1 % en poids, de 5°C à 80°C, permettent l'obtention d'une solution macroscopiquement homogène et transparente, c'est à dire ayant une valeur de transmittance maximum de la lumière, quelle que soit la longueur d'onde comprise entre 400 et 800 nm, à travers un échantillon de 1 cm d'épaisseur, d'au moins 85%, de préférence d'au moins 90 %.
Ces unités hydrosolubles ou hydrodispersibles ne présentent pas de température de démixtion par chauffage de type LCST.
Ces unités hydrosolubles ou hydrodispersibles peuvent être obtenues par polymérisation radicalaire de monomères vinyliques, ou par polycondensation, ou encore peuvent être constituées par des polymères naturels ou naturels modifiés existants.
A titre d'exemples, on peut citer les monomères hydrosolubles suivants et leurs sels, qui sont susceptibles d'être employés pour former, en tout ou partie, par polymérisation lesdites unités hydrosolubles ou hydrodispersibles, seuls ou en mélange :
- l'acide (méth)acrylique ;
- l'acide vinylsulfonique ;
- l'acide (méth)allylsulfonique ;
- l'acide vinylphosphonique ;
- le chlorure de méthyl vinylimidazolium ;
- le (méth)acrylamide ;
- la 2-vinylpyridine, la 4-vinylpyridine ;
- l'acide et l'anhydride maléiques ;
- l'acide crotonique ;
- l'acide itaconique ;
- l'alcool vinylique de formule CH₂=CHOH ;
- les N-vinyllactames comportant un groupe alkyle cyclique de 4 à 9 atomes de carbone tels que la N-vinylpyrrolidone, la N-vinylcaprolactame et la N-butyrolactame ;
- les dérivés hydrosolubles du styrène, notamment le styrène sulfonate ;
- le chlorure de diméthyldiallyl ammonium ;
- la N-vinylacétamide et la N-méthyl,N-vinylacétamide ;
- la N-vinylformamide et la N-méthyl N-vinylformamide ;
- les monomères vinyliques de formule (I) suivante : 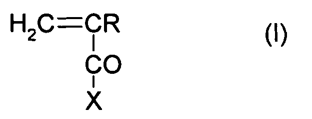 dans laquelle :
   - R est choisi parmi H, -CH₃, -C₂H₅ ou -C₃H₇, et
   - X est choisi parmi :
      - les oxydes d'alkyle de type -OR', où R' est un radical hydrocarboné, linéaire ou ramifié, saturé ou insaturé, ayant 1 à 6 atomes de carbone, substitué par au moins un groupe choisi parmi les atomes d'halogène (iode, brome, chlore, fluor); les groupements sulfonique (-SO₃⁻), sulfate (-SO₄⁻), phosphate (-PO₄H₂⁻) ; hydroxy (-OH) ; éther (-O-) ; amine primaire (-NH₂) ; amine secondaire (-NHR₁), amine tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₁ + R₂ + R₃ ne dépasse pas 7 ; et
      - les groupements -NH₂, -NHR₄ et -NR₄R₅ dans lesquels R₄ et R₅ sont indépendamment l'un de l'autre des radicaux hydrocarbonés, linéaires ou ramifiés, saturés
      ou insaturés ayant 1 à 6 atomes de carbone, sous réserve que le nombre total d'atomes de carbone de R₄ + R₅ ne dépasse pas 7, lesdits R₄ et R₅ étant substitués par au moins un groupe choisi parmi les atomes d'halogène (iode, brome, chlore, fluor) ; les groupements hydroxy (-OH) ; éther (-0-) ; sulfonique (-SO₃⁻) ; sulfate (-SO₄⁻) ; phosphate (-PO₄H₂⁻) ; amine primaire (-NH₂) ; amine secondaire (-NHR₁), tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7.
A titre d'exemples de monomères hydrosolubles de formule (I), on peut citer le (méth)acrylate de glycidyle, le méthacrylate d'hydroxyéthyle, les (méth)acrylates d'éthylène glycol, de diéthylèneglycol ou de polyalkylèneglycol, la N,N-diméthylacrylamide, l'acide acrylamido-2-méthylpropane sulfonique (AMPS), le chlorure de (méth)acrylamido propyl triméthyl ammonium (APTAC et MAPTAC) et le diméthylaminoéthylquaternisé (MADAME).

On peut citer, également parmi les monomères susceptibles d'être utilisés pour constituer, en tout ou partie, par polymérisation les unités hydrosolubles ou hydrodispersibles, des monomères hydrophobes, lesdits monomères étant copolymérisés avec au moins un monomère hydrosoluble en une quantité telle que les unités résultantes soient hydrosolubles ou hydrodispersibles, lesdits monomères hydrophobes étant choisis parmi les monomères suivants :
- le styrène et ses dérivés tels que le 4-butylstyrène, l'alpha-méthylstyrène et le vinyltoluène ;
- l'acétate de vinyle de formule CH₂=CH-OCOCH₃;
- les vinyléthers de formule CH₂=CHOR₆, dans laquelle R₆ est un radical hydrocarboné, linéaire ou ramifié, saturé ou insaturé, ayant 1 à 6 atomes de carbone ;
- l'acrylonitrile ;
- la caprolactone ;
- le chlorure de vinyle et le chlorure de vinylidène ;
- les dérivés siliconés tels que le méthacryloxypropyltris(triméthylsiloxy)silane et les méthacrylamides siliconés,
- les monomères vinyliques hydrophobes de formule (II) suivante : 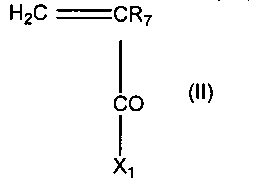 dans laquelle :
   - R₇ est choisi parmi H, -CH₃, -C₂H₅ ou C₃H₇,
   - X₁ est choisi parmi les :
      - les oxydes d'alkyle de type -OR₈ où R₈ est un radical hydrocarboné, linéaire ou ramifié, saturé ou insaturé, ayant 1 à 8 atomes de carbone ;
      - les groupements NH₂, -NHR₉ et -NR₉R₁₀ dans lesquels R₉ et R₁₀ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₉ + R₁₀ ne dépasse pas 6.

A titre d'exemples, de tels monomères, on peut citer le méthacrylate de méthyle, le méthacrylate d'éthyle, le méthacrylate de n-butyle, le méthacrylate de tertio-butyle, l'acrylate de cyclohexyle,

Les unités hydrosolubles ou hydrodispersibles peuvent être neutralisées, le cas échéant, en tout ou partie, par une base minérale ou organique choisie parmi les sels de sodium, ammonium, lithium, calcium, magnésium, ammonium substitué par 1 à 4 groupes alkyle portant de 1 à 15 atomes de carbone, ou encore parmi la mono-, la di- et la triéthanolamine, l'aminoéthylpropanediol, la N-méthyl-glucamine, les acides aminés basiques tels que l'arginine et la lysine, et leurs mélanges.
On peut noter, également, que le squelette des polymères greffés peut être réticulé par action d'au moins un agent de réticulation choisi parmi les composés à polyinsaturation oléfinique couramment utilisés pour la réticulation des polymères obtenus par polymérisation radicalaire. A titre d'exemple de tels agents, on peut citer les composés à polyinsaturation oléfinique tel que le divinylbenzène, l'éther diallylique, le dipropylèneglycol-diallyléther, les polyglycol-diallyléthers, le triéthylèneglycol-divinyléther, l'hydroquinone-diallyl-éther, le tétrallyl-oxéthanoyle, les dérivés allyl-ou vinyléthers alcools polyfonctionnels, le diacrylate de tétraéthylèneglycol, la triallylamine, le triméthylolpropanediallyléther, le tétraallyloxyéthane, le méthylène-bis-acrylamide, les éthers allyliques d'alcools de la série des sucres, le méthacrylate d'allyle, le triméthylol propane triacrylate (TMPTA) et leurs mélanges.
De préférence, on utilise un squelette de polymère greffé non réticulé.
Parmi les polycondensats et les polymères naturels ou naturels modifiés susceptibles de constituer, en tout ou partie, les unités hydrosolubles ou hydrodispersibles, on peut citer :
- les polyuréthannes hydrosolubles,
- la gomme de xanthane, notamment celle commercialisée sous les dénominations Keltrol T et Keltrol SF par Kelco, ou Rhodigel SM et Rhodigel 200 de Rhodia ;
- les alginates (Kelcosol de Monsanto) et leurs dérivés tels que l'alginate de propylèneglycol (Kelcoloid LVF de Kelco) ;
- les dérivés de cellulose et notamment la carboxyméthylcellulose (Aquasorb A500 Hercules), l'hydroxypropylcellulose, l'hydroxyéthylcellulose et l'hydroxyéthylcellulose quaternisée,
- les galactomannanes et leurs dérivés tels que la gomme Konjac, la gomme de guar, l'hydroxypropylguar, l'hydroxypropylguar modifié par des groupements méthylcarboxylate de sodium (Jaguar XC97-1, Rhodia) et le chlorure de guar hydroxypropyl triméthyl ammonium, et
- la polyéthylène-imine.

De préférence, les unités hydrosolubles ou hydrodispersibles ont une masse molaire de 1000 g/mole à 10 000 000 g/mole lorsqu'elles constituent le squelette hydrosoluble d'un polymère greffé.
Ces unités hydrosolubles ou hydrodispersibles ont, de préférence, une masse molaire de 500 g/mole à 500 000 g/mole lorsqu'elles constituent un bloc d'un polymère multiblocs ou bien lorsqu'elles constituent les greffons d'un polymère greffé.

### b) Unités à LCST

Les unités à LCST des polymères utilisés dans l'invention peuvent être définies comme des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère est appelée "LCST" (Lower Critical Solution Temperature). Pour chaque concentration en polymère, une température de démixtion par chauffage est observée ; elle est supérieure à la LCST qui est le point minimum de la courbe. En dessous de cette température, le polymère est soluble dans l'eau ; au dessus de cette température, le polymère perd sa solubilité dans l'eau.

Par soluble dans l'eau à la température T, on entend que les unités présentent une solubilité à T, d'au moins 1 g/l, de préférence d'au moins 2 g/l.
La mesure de la température de démixtion peut se faire visuellement. Par exemple, on détermine visuellement la température à laquelle apparaît le point de trouble de la solution aqueuse, dans la mesure où, à cette température, a lieu une opacification de la solution, ou perte de transparence.
D'une manière générale, la température de démixtion est déterminée par spectroscopie UV visible en mesurant, à une longueur d'onde égale à 500 nm, l'absorbance d'une solution aqueuse comprenant 1% en poids desdites unités à LSCT. La température de démixtion est identifiée à la température à laquelle l'absorbance de la solution est égale à 2.

Les unités à LCST des polymères utilisés dans l'invention peuvent être constituées d'un ou plusieurs des polymères suivants :
- les polyéthers tels que le polyoxyde d'éthylène (POE), le polyoxyde de propylène (POP), et les copolymères statistiques d'oxyde d'éthylène (OE) et d'oxyde de propylène (OP),
- le polyvinylméthyléther,
- les dérivés polymériques et copolymériques N-substitués de l'acrylamide ayant une LCST tels que le poly N-isopropylacrylamide et le poly N-éthylacrylamide,
- le polyvinylcaprolactame et les copolymères de vinyl caprolactame.

De préférence, les unités à LCST sont constituées de copolymères statistiques d'oxyde d'éthylène (OE) et d'oxyde de propylène (OP) représentés par la formule : (OE)ₘ(OP)ₙ
dans laquelle m est un nombre entier allant de 1 à 40, de préférence 2 à 20, et n est un nombre entier allant de 10 à 60, de préférence de 20 à 50.
De préférence, la masse molaire de ces unités à LCST est de 500 à 5300 g/mole, de préférence de 1500 à 4000 g/mole.
On a constaté que la répartition statistique des motifs OE et OP se traduit par l'existence d'une température inférieure critique de démixtion, au delà de laquelle une séparation de phases macroscopique est observée. Ce comportement est différent de celui des copolymères OE OP à blocs, qui micellisent au delà d'une température critique dite de micellisation (agrégation à l'échelle microscopique).

Les unités à LCST peuvent donc notamment se présenter sous la forme de copolymères statistiques d'oxyde d'éthylène et d'oxyde de propylène, aminés, notamment monoaminés, diaminés ou triaminés. Parmi les unités à LCST commercialement disponibles, on peut citer les copolymères vendus sous la dénomination Jeffamine par HUNTSMAN, et notamment la Jeffamine XTJ-507 (M-2005), la Jeffamine D-2000 et la Jeffamine XTJ-509 (ou T-3000).

Les unités à LCST peuvent également se présenter sous la forme de copolymères OE/OP statistiques à extrémités OH, tels que ceux vendus sous la dénomination Polyglycols P41 et B11 par Clariant.

Avantageusement, on peut utiliser selon l'invention des polymères présentant des unités à LCST, tels que les dérivés polymériques et copolymériques N-substitués de l'acrylamide ayant une LCST.
A titre d'exemples de dérivés N-substitués de l'acrylamide, on peut citer le poly N-isopropyl acrylamide, le poly N-éthyl acrylamide et les copolymères de N-isopropyl acrylamide ou de N-éthyl acrylamide et d'un monomère vinylique répondant à la formule (I) ou (II) donnée ci-dessus, ou d'un monomère choisi parmi l'acide (méth)acrylique, l'acide vinylsulfonique, l'acide (méth)allylsulfonique, l'acide et l'anhydride maléique, l'acide vinylphosphonique, l'acide crotonique, l'acide itaconique, la (méth)acrylamide, la vinylpyridine, l'alcool vinylique, les N-vinyllactames tels que la N-vinylpyrrolidone, le styrène et ses dérivés, le chlorure de diméthyldiallyl ammonium, la N-vinylacétamide, la N-méthyl N-vinylacétamide, la N-vinylformamide, la N-méthylvinylformamide, les vinyléthers , l'acétate de vinyle, l'acrylonitrile, la caprolactone, le chlorure de vinyle, le chlorure de vinylidène.

La masse molaire des polymères et copolymères N-substitués de l'acrylamide est, de préférence, de 1000 g/mole à 500 000 g/mole.
La synthèse de ces polymères peut être réalisée par polymérisation radicalaire à l'aide d'un couple d'amorceurs tel que le chlorhydrate d'aminoéthanethiol en présence de persulfate de potassium afin d'obtenir des oligomères ayant une extrémité réactive aminée.

A titre d'exemples de copolymères de N-vinylcaprolactame, on peut citer les copolymères de N-vinylcaprolactame et d'un monomère vinylique répondant à la formule (I) ou (II) donnée ci-dessus, ou d'un monomère choisi parmi l'acide (méth)acrylique, l'acide vinylsulfonique, l'acide (méth)allylsulfonique, l'acide maléique, l'anhydride maléique, l'acide vinylphosphonique, l'acide crotonique, l'acide itaconique, la (méth)acrylamide, la vinylpyridine, l'alcool vinylique, les N-vinyllactames tels que la N-vinylpyrrolidone, le styrène et ses dérivés, le chlorure de diméthyldiallylammonium, la N-vinylacétamide, la N-méthyl-N-vinylacétamide, la N-vinylformamide, la N-méthylvinylformamide, l'acétate de vinyle, les vinyléthers, l'acrylonitrile, la caprolactone, le chlorure de vinyle et le chlorure de vinylidène.
La masse molaire de ces polymères est de préférence de 1000 g/mole à 500 000 g/mole.
La synthèse de ces polymères peut être réalisée par polymérisation radicalaire à l'aide d'un couple d'amorceurs tel que le chlorhydrate d'aminoéthanethiol en présence d'azobisisobutyronitrile afin d'obtenir des oligomères ayant une extrémité réactive aminée.

La proportion massique des unités à LCST dans le polymère final est de préférence de 5 à 70 %, notamment de 20 à 65%, et particulièrement de 30 à 60 % en poids par rapport au polymère final.
Comme défini précédemment, la température de démixtion par chauffage des unités à LCST est de 5 à 40 °C, de préférence, de 10 à 35°C pour une concentration massique des unités à LCST dans l'eau de 1%.

Les polymères utilisés dans le cadre de cette invention peuvent être aisément préparés par l'homme du métier par différentes méthodes, telles que les procédés de greffage, de copolymérisation, de couplage ou de polymérisation vivante.
Par exemple, lorsque le polymère final se présente sous la forme d'un polymère greffé présentant notamment un squelette hydrosoluble avec des unités latérales à LCST, il est possible de le préparer par greffage des unités à LCST ayant au moins une extrémité réactive, par exemple aminée, sur un polymère hydrosoluble formant le squelette, ledit squelette portant au minimum 10% (en mole) de groupes réactifs tels que des fonctions acides carboxyliques. Cette réaction peut s'effectuer en présence d'un carbodiimide tel que le dicyclohexylcarbodiimide ou le chlorohydrate de 1-(3-diméthylaminopropyl)-3-éthylcarbodiimide, dans un solvant tel que la N-méthylpyrrolidone ou l'eau.
Une autre possibilité pour préparer des polymères greffés consiste à copolymériser par exemple un macromonomère à LCST (unité précédemment décrite avec une extrémité insaturée) et un monomère vinylique hydrosoluble tel que l'acide acrylique ou les monomères vinyliques ayant la formule (I) citée précédemment.
Lorsque le polymère final se présente sous la forme d'un polymère bloc, il est possible de le préparer par couplage entre des unités hydrosolubles ou hydrodispersibles et des unités à LCST ayant à chaque extrémité des sites réactifs complémentaires.
Il est possible également de préparer de tels polymères par polymérisation vivante de type anionique ou cationique, ou bien par polymérisation radicalaire contrôlée. Ce dernier procédé de synthèse peut être mis en oeuvre par différentes méthodes comme la méthode par transfert d'atomes (Atom Transfert Radical Polymerization ou ATRP), la méthode des radicaux tels que les nitroxydes ou encore par la méthode par transfert de chaîne réversible avec addition-fragmentation (Radical Addition-Fragmentation Chain Transfert) telle que le procédé MADIX (Macromolecular Design via the Interchange of Xanthate). Ces procédés peuvent être utilisés pour obtenir les blocs hydrosolubles et les blocs à LCST des polymères utilisés dans le cadre de l'invention. Ils peuvent être utilisés également pour synthétiser un seul des deux types de blocs du polymère utilisé selon l'invention, l'autre bloc étant introduit dans le polymère final par l'intermédiaire de l'amorceur utilisé ou bien par réaction de couplage entre les blocs hydrosolubles et à LCST.

Le polymère peut être présent dans la composition selon l'invention en une teneur en matières sèches allant de 0,01% à 20% en poids, par rapport au poids total de la composition, de préférence de 0,1 à 15% en poids par rapport au poids total de la composition.

### Milieu aqueux physiologiquement acceptable

Le milieu aqueux de la composition selon l'invention peut être constitué essentiellement d'eau (notamment le milieu aqueux est de l'eau) ou bien encore peut comprendre un mélange d'eau et d'un ou plusieurs solvants miscible(s) à l'eau, comme les monoalcools inférieurs ayant de 1 à 5 atomes de carbone, tels que l'éthanol, les glycols ayant de 2 à 8 atomes de carbone, les cétones en C₃-C₄ et les aldéhydes en C₂-C₄.

La teneur en milieu aqueux de la composition peut aller de 5 à 95 % en poids par rapport au poids total de la composition, de préférence de 20 à 80% en poids.

La composition selon l'invention peut comprendre, outre le polymère à unités LCST, un agent épaississant, en particulier un agent épaississant de milieu aqueux, tels que les argiles hydrophiles comme les hectorites, les bentonites, comme par exemple la Laponite XLS fournie par la société Rockwood, les épaississants associatifs tels que les polyuréthanes associatifs comme le Serad FX commercialisé par la société Servo, les polymères acryliques associatifs, les épaississants cellulosiques hydrosolubles tels que l'hydroxyéthylcellulose, les gommes naturelles, telle que la gomme xanthane.

L'agent épaississant peut être présent en une teneur allant de 0,05 % à 5 % en poids, par rapport au poids total de la composition

La composition peut en outre comprendre au moins un polymère filmogène (polymère additionnel) en une teneur en matières sèches (ou matière active) allant de 5% à 70% en poids par rapport au poids total de la composition, mieux de 10 à 60% en poids et encore mieux de 20% à 50%.

Dans la présente demande, on entend par « polymère filmogène » un polymère apte à former à lui seul ou en présence d'un agent plastifiant éventuel, un film isolable. Le polymère filmogène peut être solubilisé ou dispersé sous forme de particules dans le milieu cosmétiquement acceptable de la composition.

Le polymère filmogène peut être choisi parmi les polymères radicalaires obtenus à partir de monomères comprenant au moins une insaturation, les polycondensats et les polymères d'origine naturelle.

Le polymère filmogène peut être choisi notamment dans le groupe formé par les polymères vinyliques et les acryliques, les polyuréthanes, les polyesters, les résines alkydes, les résines époxyesters, les polymères cellulosiques, tels que la nitrocellulose, les esters de cellulose comme l'acétate de cellulose, l'acétopropionate de cellulose, l'acétobutyrate de cellulose, les résines résultant de la condensation de formaldéhyde avec une arylsulfonamide et leurs mélanges.

Le polymère filmogène est généralement présent sous la forme de particules en dispersion dans le milieu aqueux de la composition, formant ainsi un latex ou pseudo latex. Les particules de polymère ont généralement une taille allant de 10 à 200nm, de préférence allant de 20 à 150 nm et mieux de 50 à 100 nm.

Parmi les polymères filmogènes susceptibles d'être utilisés dans la composition selon l'invention, on peut citer les polyuréthanes, par exemple anioniques, les polyesters-polyuréthanes, les polyéther-polyuréthanes, les polymères radicalaires, notamment de type acrylique, acrylique styrène et/ou vinylique, les polyesters, les résines alkydes, seuls ou en mélange.

Le(s) polymère(s) filmogène(s) en dispersion aqueuse peut être choisi parmi les dispersions aqueuses de polymères et/ou de copolymères acryliques, acryliques/ styrène et vinyliques, présentant un taux de matière sèche allant de 30 à 50%.
Les polymères filmogènes acryliques utilisables selon l'invention peuvent résulter de la polymérisation d'au moins un monomère à insaturation éthylénique choisis parmi les acides carboxyliques-éthyléniques, leurs esters et leurs amides. Comme acide carboxylique insaturé -éthyléniques, on peut utiliser l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique. Les esters de ces acides carboxyliques peuvent être choisis parmi les esters de l'acide (méth)acrylique (encore appelé les (méth)acrylates), notamment des (méth)acrylates d'alkyle, en particulier d'alkyle en C₁-C₃₀, de préférence en C₁-C₂₀, des (méth)acrylates d'aryle, en particulier d'aryle en C₆-C₁₀, des (méth)acrylates d'hydroxyalkyle, en particulier d'hydroxyalkyle en C₂-C₆. Parmi les (méth)acrylates d'alkyle, on peut citer le méthacrylate de méthyle, le méthacrylate d'éthyle, le méthacrylate de butyle, le méthacrylate de d'isobutyle, le méthacrylate d'étyle-2 hexyle, le méthacrylate de lauryle, le méthacrylate de cyclohexyle. Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle. Il est possible bien entendu d'employer un mélange de ces monomères. Les esters de l'acide (méth)acrylique particulièrement préférés sont les (méth)acrylates d'alkyle. Selon la présente invention, le groupement alkyle peut être soit fluoré, soit perfluoré, c'est à dire qu'une partie ou la totalité des atomes d'hydrogènes du groupement alkyle sont substitués par des atomes de fluor.
Comme amides desdits acides carboxyliques, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-butyl acrylamide, le N-octyl acrylamide et le N-undécylacrylamide.
Le polymère filmogène acrylique utilisable selon l'invention peut comprendre, en plus des monomères cités précédemment, au moins un monomère styrénique, tel que le styrène ou l'alpha-méthyl styrène.
Comme polymère acrylique en dispersion aqueuse, on peut citer les dispersions de polymères acryliques vendues sous les dénominations NEOCRYL XK-90®, NEOCRYL A-1070®, NEOCRYL A-1090®, NEOCRYL BT-62®, NEOCRYL A-1079®, NEOCRYL A-523®, NEOCRYL XK63 par la Société Avecia Resins, DOW LATEX 432®, par la Société DOW CHEMICAL, JONCRYL77 ou JONCRYL90 par la Société Johnson, LUHYDRAN A848S » par la Société BASF, SYNTRAN 5190 par la Société INTERPOLYMER.

Le(s) polymère(s) filmogène(s) en dispersion aqueuse peut être choisi parmi les dispersions aqueuses de polyuréthane anionique, de polyester-polyuréthane, de polyéther-polyuréthane et leur mélange, qui présentent de préférence un taux de matière sèche allant de 20 à 40%. La dispersion aqueuse de polyuréthanne peut être, par exemple, une dispersion aqueuse de polyuréthanne anionique, de polyester-polyuréthanne et/ou de polyether-polyuréthane, seul ou en mélange, pouvant présenter un taux de matière sèche de 20 à 40%.

A titre de dispersions aqueuses de polyuréthane, on peut citer les polyester-polyuréthanes vendus sous les dénominations «AVALURE UR-405®», «AVALURE UR-410®», « AVALURE UR-425® », « SANCURE 2060® » par la Société GOODRICH, les polyéther-polyuréthanes vendus sous les dénominations « SANCURE 878® » par la Société GOODRICH, « NEOREZ R-970® » par la Société AVECIA, ou encore les dispersions aqueuse de polyuréthane obtenu par polycondensation du diisocyanate de tétraméthylxylylène telles que le « Cydrothane HP 1035 », le « Cydrothane HP 4033 », le « Cydrothane HP 5035 » et le « Cydrothane HP 6000 » de la société Cytec ou encore les dispersions commercialisés sous la dénomination « IW/01.1, IW/019.1, IW/028.1 » par la société UCB.

Pour améliorer les propriétés filmogènes de la composition de vernis à ongle un agent auxiliaire de filmification peut être prévu.
Un tel agent auxiliaire de filmification peut être choisi parmi tous les composés connus de l'homme du métier, comme étant susceptibles de remplir la fonction recherchée, et être notamment choisi parmi les agents plastifiants, les agents de coalescence et leur mélange.

Comme agent plastifiant, on peut citer, seuls ou en mélange, les plastifiants usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les esters de glycol ;
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, des tartrates, des phosphates, des sébaçates ;
- des dérivés oxyéthylénés, tels que les huiles oxyéthylénées, notamment les huiles végétales, telles que l'huile de ricin ;
- leurs mélanges.

La quantité de plastifiant peut être choisie par l'homme du métier sur base de ses connaissances générales, de manière à obtenir une composition ayant des propriétés cosmétiquement acceptables. La teneur en plastifiant peut, par exemple, aller de 0,1 % à 15 % en poids, par rapport au poids total de la composition, et de préférence de 0,5 % à 10 % en poids.

La composition peut comprendre une matière colorante qui peut être choisie parmi les composés pulvérulents et/ou les colorants hydrosolubles. La matière colorante peut être présente en une teneur allant de 0,01 % à 50 % en poids par rapport au poids total de la composition, de préférence de 0,05 à 30% en poids et mieux de 0,1 à 20% en poids.

Les composés pulvérulents peuvent être choisis parmi les pigments et/ou les nacres et/ou les paillettes, habituellement utilisés dans les vernis à ongles.
Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique, les pigments métalliques comme l'aluminium, le cuivre ou le bronze. Parmi les pigments organiques, on peut citer le noir de carbone, les pigments de type D & C, et les laques à base de carmin de cochenille, de baryum, strontium, calcium, aluminium, la guanine.

On peut également citer les pigments à effet tels les particules comportant un substrat organique ou minéral, naturel ou synthétique, par exemple le verre, les résines acrylique, polyester, polyuréthane, polyéthylène téréphtalate, les céramiques, les alumines recouvertes ou non de substances métalliques comme l'aluminium, l'or, le cuivre, le bronze, ou d'oxydes métalliques comme le dioxyde de titane, l'oxyde de fer, l'oxyde de chrome, de pigments minéraux ou organiques et leurs mélanges.

Les pigments nacrés peuvent être choisis parmi les pigments nacrés blancs, tels que le mica recouvert de titane, ou d'oxychlorure de bismuth, les pigments nacrés colorés, tels que le mica titane avec des oxydes de fer, le mica titane avec notamment du bleu ferrique ou de l'oxyde de chrome, le mica titane avec un pigment organique du type précité ainsi que les fluorophlogopite avec des oxydes de fer.

On peut aussi utiliser des pigments à propriétés goniochromatiques, notamment à cristaux liquides ou multicouches.

Les colorants hydrosolubles sont, par exemple, le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre, en outre, tout autre ingrédient, additif, utilisé couramment dans les compositions cosmétiques et connu de l'homme du métier comme étant susceptible d'être incorporé dans une composition de vernis à ongles.

De tels ingrédients, additifs, peuvent être choisis parmi les agents de coalescence, les agents épaississants (tels que les argiles hydrophiles, les polyuréthanes associatifs, les acides polyacryliques associatifs, la cellulose et les dérivés cellulosiques, les gommes et leurs mélanges), les conservateurs, les parfums, les huiles, les cires, les tensioactifs, les antioxydants, les agents antiradicaux libres, les agents d'étalement, les agents mouillants, les agents dispersants, les anti-mousse, les neutralisants, les stabilisants, les actifs choisis parmi les huiles essentielles, les filtres UV/solaires organiques et minéraux, les parfums, les agents hydratants, les vitamines, les protéines, les céramides , les extraits végétaux, etc.. ; et leurs mélanges.

De préférence, la composition de vernis à ongles selon l'invention est exempte de phase huileuse comprenant au moins une huile de type alcanes, esters, triglycérides, éthers, siliconées et fluorées.

Bien entendu, l'homme du métier veillera à choisir ce ou ces éventuels composés complémentaires et/ou leur quantité, de manière telle que les propriétés de la composition selon l'invention ne soient pas, ou substantiellement pas altérées par l'adjonction envisagée.

Les exemples qui suivent illustrent de manière non limitative l'invention. Sauf indications contraire, les quantités indiquées sont exprimées en gramme

### Exemple 1 : Préparation d'un polymère à unités hydrosolubles et à unités LCST

On prépare un polymère constitué par un squelette hydrosoluble d'acide polyacrylique (PAA) 450 000 g/mole portant 3,9% (en mole) des chaînes latérales ou greffons à LCST qui sont des copolymère statistique d'oxyde d'éthylène (6) et d'oxyde de propylène (39) (Jeffamine M-2005 de PM 2600).

Dans un réacteur de 500 ml muni d'un réfrigérant, on dissout 3 grammes d'acide polyacrylique de masse molaire moyenne 450 000 g/mole (Aldrich) dans 220 ml de N-méthyl pyrrolidone sous agitation à 60°C pendant 12 heures.
On dissout 4,181 grammes de copolymère (OE)₆(OP)₃₉ statistique mono-aminé, de masse molaire 2600 g/mole ayant un point de trouble, à une concentration de 1% en poids dans l'eau, de 16°C (Jeffamine M-2005 de Huntsman) dans 50 ml de N-méthylpyrrolidone sous agitation, à 20°C, pendant 15 minutes. La solution obtenue est ajoutée goutte à goutte au milieu réactionnel contenant l'acide polyacrylique, sous vive agitation à 60°C.
On dissout 2,158 grammes de dicyclohexylcarbodiimide dans 30 ml de N-méthylpyrrolidone sous agitation à 20°C pendant 15 minutes. La solution obtenue est ajoutée goutte à goutte au milieu réactionnel contenant l'acide polyacrylique et le copolymère (OE)₆(OP)₃₉ statistique mono-aminé, sous vive agitation à 60°C. On agite le mélange final pendant 12 heures à 60°C.
On refroidit le mélange à 20°C puis on le place dans un réfrigérateur à 4°C pendant 24 heures. Les cristaux de dicyclohexylurée formés sont éliminés par filtration du milieu réactionnel.
Le polymère est alors neutralisé à l'aide de 19 g de soude à 35% (4 fois en excès par rapport au nombre de mole d'acide acrylique), ce qui conduit à sa précipitation. Après 12 heures au repos, le milieu réactionnel est filtré afin de récupérer le polymère précipité. Celui-ci est séché sous vide à 35°C pendant 24 heures.
On récupère 13,55 grammes de solide qui sont dissous dans 2 litres d'eau désionisée. Cette solution est ultrafiltrée à l'aide d'un système d'ultrafiltration Millipore contenant une membrane dont le seuil de coupure est fixée à 10 000 Daltons. La solution ainsi purifiée est lyophilisée afin de recueillir le polymère sous forme solide.
On obtient 7,05 grammes d'acide polyacrylique (450 000 g/mole) greffé par 3,9% (en mole) de copolymère (OE)₆(OP)₃₉ statistique mono-aminé.
La proportion massique des unités à LCST dans le polymère final est de 51 %.

Le polymère obtenu est caractérisé par la masse molaire du squelette hydrosoluble (acide polyacrylique), la nature chimique des chaînes à LCST, leur proportion massique dans le polymère et leur masse molaire comme suit :

| | Squelette hydrosoluble | Greffons (unités à LCST) | Proportion : unités à LCST dans polymère final (en poids) | Taux de greffage (% en mole) |
|---|---|---|---|---|
| Polymère | Acide polyacrylique; PM=450000 | (OE)₆(OP)₃₉ statistique Jeffamine M-2005; PM=2600 | 51 % | 3,9% |

Ce polymère présente les caractéristiques suivantes :

| | |
|---|---|
| Température de démixtion des greffons à 1% dans l'eau : | 23°C |
| Température de gélification du polymère à 2% dans l'eau | 28°C |
| Concentration critique d'agrégation dans l'eau du polymère | 0,9%. |

La température de démixtion des unités Jeffamine est déterminée par spectroscopie UV visible en mesurant, à une longueur d'onde égale à 500 nm, l'absorbance d'une solution aqueuse comprenant 1% en poids desdites unités à LSCT. La température de démixtion est identifiée à la température à laquelle l'absorbance de la solution est égale à 2.

La température de gélification d'une solution aqueuse d'un polymère de l'invention est déterminée par des mesures de rhéologie : il s'agit de la température à partir de laquelle la viscosité de la solution du polymère devient supérieure à la viscosité d'une solution du polymère équivalent ne comportant pas d'unités à LCST.
La température de gélification d'une solution à 2% de polymère dans l'eau correspond à la température de gélification atteinte lorsque la différence entre la viscosité de la solution de polymère et la viscosité d'une solution du polymère équivalent ne comportant pas d'unités à LCST, est supérieure à 5%. La viscosité de la composition est mesurée à l'aide d'un rhéomètre Haake RS150 muni d'une géométrie cône/plan 3,5 cm/2° ou 6 cm/2° et d'un système de contrôle de la température. Les mesures de viscosité sont réalisées dans le mode écoulement en imposant un gradient de cisaillement égal à 10⁻¹ s⁻¹.

La concentration d'agrégation critique (CAC) du polymère est determinée dans l'eau pure par rhéologie. Il s'agit de la concentration à partir de laquelle la viscosité d'une solution aqueuse du polymère considéré devient supérieure à la viscosité d'une solution du polymère équivalent ne comportant pas d'unités à LCST. La mesure de viscosité est réalisée à l'aide d'un rhéomètre Haake RS150 équipé d'une géométrie cône/plan (35 mm, 2°) et d'un bain thermostaté afin de contrôler la température entre 5 et 80°C. Les mesures ont été effectuées dans le mode écoulement à une vitesse de cisaillement de 10 s⁻¹, en faisant varier la température de 15°C à 50°C à une vitesse de 0,5°C/minute.

### Exemple 2 et 3 : Vernis à ongles de teinte blanche

On a réalisé deux vernis à ongles de teinte blanche :
- le vernis de l'exemple 2 selon l'invention comprend le polymère gélifiant de l'exemple 1,
- le vernis à ongles de l'exemple 3 (comparatif) ne comprend pas de polymère à unités hydrosolubles et à unités LCST et comprend un polyuréthane associatif (Serad FX 1100).

| | **Exemple 2 Invention** | **Exemple 3 comparatif** |
|---|---|---|
| - Polymère de l'exemple 1 | 0,5 | |
| - Polyuréthane associatif vendu sous la dénomination « Serad FX 1100 » par la société Servo Hüls | | 0,08 |
| Dispersion aqueuse de polyuréthane à 35% en poids de matières sèches vendue sous la dénomination « Cydrothane HP 6000» par la société Cytec | 30,9 (MA) | 30,9 (MA) |
| Colorant inorganique blanc vendu sous la dénomination « Blanc Covarine W 9797 » par la société Wackherr | 1,6 | 1,6 |
| Diazolidinyl urée | 0,39 | 0,39 |
| Perfluorokexyl bétaïne | 0,05 | 0,05 |
| Ethanol | 4,25 | 4,25 |
| Conservateurs | 0,54 | 0,54 |
| Eau déminéralisée | Qsp 100 | Qsp 100 |

### Evaluation de l'homogénéité de couleur

Ces vernis sont placés deux mois à 45°C :
Le vernis de l'exemple 2 présente un bonne homogénéité de couleur dans le temps : après avoir été placé deux mois à 45°C (température supérieure à la température de gélification du polymère polyacrylate de sodium à greffons Jeffamine qui est de 28°C), il reste blanc depuis le haut jusqu'au bas du flacon le contenant.
Le vernis de l'exemple 3 présente une hétérogénéité en couleur (apparition d'un surnageant translucide en haut du flacon le contenant).

On met en évidence le pouvoir gélifiant du polymère en mesurant la viscosité de la composition de vernis à ongles ci-dessus :
Les mesures de viscosité ont été réalisées à l'aide d'un rhéomètre Haake RS150 équipé d'une géométrie cône/plan (35 mm, 2°) et d'un bain thermostaté afin de contrôler la température entre 4°C et 80°C. Les mesures ont été effectuées dans le mode écoulement, à une vitesse de cisaillement imposée égale à 10 s⁻¹, en faisant varier la température de 4°C à 45°C à la vitesse de 0,5°C/minute.

| | **Exemple 2** | **Exemple 3** |
|---|---|---|
| **Viscosité à 20°C (10 s**^{**-1**}**)** | 0,08 Pa.s | 0,08 Pa.s |
| **Viscosité à 35°C (10 s**^{**-1**}**)** | 0,15 Pa.s | 0,04 Pa.s |

La viscosité du vernis de l'exemple 2 augmente lorsque la température est supérieure à la température de gélification du polymère.
La viscosité de la composition 3, qui ne comprend pas de polymère à LCST, diminue lorsque la température augmente.

### Exemple 4 et 5 : Vernis à ongles de teinte rose

On a réalisé deux vernis à ongles de teinte rose :
- le vernis de l'exemple 4 selon l'invention comprend le polymère gélifiant de l'exemple 1
- le vernis à ongles de l'exemple 5 (comparatif) ne comprend pas de polymère à unités hydrosolubles et à unités LCST et comprend un polyuréthane associatif (Serad FX 1100).

Les compositions sont données dans le tableau suivant :

| | **Exemple 4 Invention** | **Exemple 5 comparatif** |
|---|---|---|
| - Polymère de l'exemple 1 | 0,5 | |
| - Polyuréthane associatif vendu sous la dénomination « Serad FX 1100 » par la société Servo Hüls | | 0,08 |
| Dispersion aqueuse de polyuréthane à 35% en poids de matières sèches vendue sous la dénomination « Cydrothane HP 6000» par la société Cytec | 30,9 (MA) | 30,9 (MA) |
| Colorant inorganique blanc vendu sous la dénomination « Blanc Covarine W 9797 » par la société Wackherr | 0,8 | 0,8 |
| Colorant organique rouge vendu sous la dénomination « Rouge Covarine W 3799 » par la société Wackherr | 1,2 | 1,2 |
| Diazolidinyl urée | 0,39 | 0,39 |
| Perfluorohexyl bétaïne | 0,05 | 0,05 |
| Ethanol | 4,25 | 4,25 |
| Conservateurs | 0,54 | 0,54 |
| Eau déminéralisée | Qsp 100 | Qsp 100 |

### Evaluation de l'homogénéité de couleur

Ces vernis sont placés deux mois à 45°C :
Le vernis de l'exemple 4 présente un bonne homogénéité de couleur dans le temps : après avoir été placé deux mois à 45°C, il reste rose depuis le haut jusqu'au bas du flacon le contenant.
Le vernis de l'exemple 5 présente une hétérogénéité en couleur après 2 mois à 45°C : il présente d'importantes marbrures blanches le long de la paroi du flacon le contenant.

### Exemple 6 et 7 : Vernis à ongles de teinte verte

On a réalisé deux vernis à ongles de teinte verte :
- le vernis de l'exemple 6 selon l'invention selon l'invention comprend le polymère gélifiant de l'exemple 1,
- le vernis à ongles de l'exemple 7 (comparatif) ne comprend pas de polymère à unités hydrosolubles et à unités LCST et comprend un polyuréthane associatif (Serad FX 1100).

| | **Exemple 6 Invention** | **Exemple 7 comparatif** |
|---|---|---|
| - Polymère de l'exemple 1 | 0,5 | |
| - Polyuréthane associatif vendu sous la dénomination « Serad FX 1100 » par la société Servo Hüls | | 0,08 |
| Dispersion aqueuse de polyuréthane à 35% en poids de matières sèches vendue sous la dénomination « Cydrothane HP 6000» par la société Cytec | 30,9 (MA) | 30,9 (MA) |
| Colorant organique bleu vendu sous la dénomination « Bleu Covarine W 6795 » par la société Wackherr | 1,2 | 1,2 |
| Colorant organique jaune vendu sous la dénomination « Jaune Covarine W 1796 » par la société Wackherr | 1,2 | 1,2 |
| Diazolidinyl urée | 0,39 | 0,39 |
| Perfluorokexyl bétaïne | 0,05 | 0,05 |
| Ethanol | 4,25 | 4,25 |
| Conservateurs | 0,54 | 0,54 |
| Eau déminéralisée | Qsp 100 | Qsp 100 |

### Evaluation de l'homogénéité de couleur

Ces vernis sont placés deux mois à 45°C :
Le vernis de l'exemple 6 présente un bonne homogénéité de couleur dans le temps : après avoir été placé deux mois à 45°C, il reste rose depuis le haut jusqu'au bas du flacon le contenant.
Le vernis de l'exemple 7 présente une hétérogénéité en couleur : il présente une couche de couleur jaune dans le bas du flacon le contenant.

## Revendications

1. Composition de vernis à ongles comprenant, dans un milieu aqueux cosmétiquement acceptable, un polymère hydrosoluble ou hydrodispersible, le polymère étant tel que la viscosité de ladite composition augmente lorsqu'elle est soumise à une température supérieure à la température de gélification dudit polymère.

2. Composition selon la revendication précédente, **caractérisée en ce que** le polymère est tel que, lorsque la composition est soumise à une température supérieure ou égale à la température de gélification dudit polymère + 20°C, la viscosité de ladite composition augmente d'au moins 10% par rapport à sa viscosité mesurée à la température de gélification du polymère, pour une vitesse de cisaillement égale à 10 s⁻¹.

3. Composition selon la revendication 1 ou 2, **caractérisée en ce que** le polymère hydrosoluble ou hydrodispersible comprend des unités hydrosolubles ou hydrodispersibles et des unités à LCST, lesdites unités à LCST présentant dans l'eau une température de démixtion de 5 à 40 °C à une concentration massique de 1 %.

4. Composition de vernis à ongles comprenant, dans un milieu aqueux cosmétiquement acceptable, un polymère hydrosoluble ou hydrodispersible comprenant des unités hydrosolubles ou hydrodispersibles et des unités à LCST, lesdites unités à LCST présentant dans l'eau une température de démixtion de 5 à 40 °C à une concentration massique de 1 %.

5. Composition selon l'une des revendications précédentes, dans laquelle le polymère présente une température de gélification de 5 à 50°C pour une concentration massique dans l'eau de 2 %.

6. Composition selon l'une des revendications précédentes, dans laquelle le polymère se présente sous la forme d'un polymère séquencé (ou blocs) comprenant des unités hydrosolubles ou hydrodispersibles et des unités à LCST alternées ou sous la forme d'un polymère greffé constitué d'un squelette éventuellement réticulé formé d'unités hydrosolubles ou hydrodispersibles et porteur de greffons constitués d'unités à LCST ou vice-versa.

7. Composition selon l'une quelconque des revendications 3 à 6, dans laquelle les unités hydrosolubles ou hydrodispersibles du polymère, en tout ou partie, sont obtenues par polymérisation d'au moins un monomère hydrosoluble ou son sel choisi parmi :
- l'acide (méth)acrylique ;
- l'acide vinylsulfonique ;
- l'acide (meth)allylsulfonique ;
- l'acide vinylphosphonique ;
- le chlorure de méthyl vinylimidazolium ;
- le (méth)acrylamide ;
- la 2-vinylpyridine, la 4-vinylpyridine ;
- l'acide et l'anhydride maléiques ;
- l'acide crotonique ;
- l'acide itaconique ;
- l'acide vinylique de formule CH₂=CHOH ;
- les N-vinyllactames comportant un groupe alkyle cyclique de 4 à 9 atomes de carbone tels que la N-vinylpyrrolidone, la N-vinylcaprolactame et la N-butyrolactame ;
- les dérivés hydrosolubles du styrène, notamment le styrène sulfonate ;
- le chlorure de diméthyldiallyl ammonium ;
- la N-vinylacétamide et la N-méthyl,N-vinylacétamide ;
- la N-vinylformamide et la N-méthyl N-vinylformamide.
- les monomères vinyliques de formule (I) suivante : 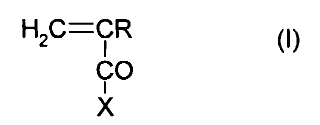 dans laquelle :
- R est choisi parmi H, -CH₃, -C₂H₅ ou -C₃H₇, et
- X est choisi parmi :
- les oxydes d'alkyle de type -OR' où R' est un radical hydrocarboné, linéaire ou ramifié, saturé ou insaturé, ayant de 1 à 6 atomes de carbone, substitué par au moins un groupe choisi parmi les atomes d'halogène (iode, brome, chlore, fluor); les groupements sulfonique (-SO₃⁻), sulfate (-SO₄⁻), phosphate (-PO₄H₂⁻) ; hydroxy (-OH) ; éther (-O-) ; amine primaire (-NH₂) ; amine secondaire (-NHR₁), tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₁ + R₂ + R₃ ne dépasse pas 7 ; et
- les groupements -NH₂, -NHR₄ et -NR₄R₅ dans lesquels R₄ et R₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄ + R₅ ne dépasse pas 7, lesdits R₄ et R₅ étant substitués par au moins un groupe choisi parmi les atomes d'halogène (iode, brome, chlore, fluor) ; les groupements hydroxy (-OH) ; sulfonique (-SO₃⁻) ; sulfate (-SO₄⁻) ; phosphate (-PO₄H₂⁻) ; amine primaire (-NH₂) ; amine secondaire (NHR₁), tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7 ;

8. Composition selon l'une quelconque des revendications 3 à 7, dans laquelle les unités hydrosolubles ou hydrodispersibles du polymère, en tout ou partie, sont obtenues par copolymérisarion d'au moins un monomère hydrophobe avec au moins un monomère hydrosoluble, ledit monomère hydrophobe étant en quantité telle que les unités résultantes soient hydrosolubles ou hydrodispersibles, ledit monomère hydrophobe étant choisi parmi :
- le styrène et ses dérivés tels que le 4-butylstyrène, l'alpha-méthylstyrène et le vinyltoluène ;
- l'acétate de vinyle de formule CH₂=CH-OCOCH₃ ;
- les vinyléthers de formule CH₂=CHOR₆, dans laquelle R₆ est un radical hydrocarboné, linéaire ou ramifié, saturé ou insaturé, ayant de 1 à 6 atomes de carbone ;
- l'acrylonitrile ;
- la caprolactone ;
- le chlorure de vinyle et le chlorure de vinylidène ;
- les dérivés siliconés tels que le méthacryloxypropyltris(triméthylsiloxy)silane, les méthacrylamides siliconés,
- les monomères vinyliques hydrophobes de formule (II) suivante : 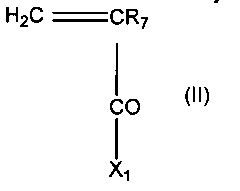 dans laquelle :
- R₇ est choisi parmi H, -CH₃, -C₂H₅ ou C₃H₇,
- X₁ est choisi parmi:
- les oxydes d'alkyle de type -OR₈ où R₈ est un radical hydrocarboné, linéaire ou ramifié, saturé ou insaturé, ayant de 1 à 8 atomes de carbone ;
- les groupements NH₂, -NHR₉ et -NR₉R₁₀ dans lesquels R₉ et R₁₀ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₉ + R₁₀ ne dépasse pas 6.

9. Composition selon la revendication 7 ou 8, dans laquelle les unités hydrosolubles ou hydrodispersibles du polymère sont neutralisées, en tout ou partie par une base minérale ou organique choisie parmi les sels de sodium, ammonium, lithium, calcium, magnésium, ammonium substitué par 1 à 4 groupes alkyle portant de 1 à 15 atomes de carbone, la mono-, la di- et la triéthanolamine, l'aminoéthylpropanediol, la N-méthyl-glucamine, les acides aminés basiques tels que l'arginine et la lysine, et leurs mélanges.

10. Composition selon l'une quelconque des revendications 7 à 9, dans laquelle les unités hydrosolubles ou hydrodispersibles du polymère sont réticulées par action d'au moins un composé à polyinsaturation oléfinique choisi parmi le divinylbenzène, l'éther diallylique, le dipropylèneglycol-diallyléther, les polyglycol-diallyléthers, le triéthylèneglycol-divinyléther, l'hydroquinone-diallyl-éther, le tétrallyl-oxéthanoyle, les dérivés allyl-ou vinyléthers d'alcools polyfonctionnels, le diacrylate de tétraéthylèneglycol, la triallylamine, le triméthylolpropanediallyléther, le tétraallyloxyéthane, le méthylène-bis-acrylamide, les éthers allyliques d'alcools de la série des sucres, le méthylène-bis-acrylamide, le méthacrylate d'allyle, le triméthylol propane triacrylate (TMPTA), et leurs mélanges.

11. Composition selon l'une quelconque des revendications 3 à 6, dans laquelle les unités hydrosolubles ou hydrodispersibles du polymère sont constituées en tout ou en partie d'un ou plusieurs des composants suivants :
- les polyuréthannes hydrosolubles,
- la gomme de xanthane,
- les alginates et leurs dérivés tels que l'alginate de propylèneglycol,
- les dérivés de cellulose et notamment la carboxyméthylcellulose, l'hydroxypropylcellulose, l'hydroxyéthylcellulose et l'hydroxyéthylcellulose quaternisée,
- les galactomannanes et leurs dérivés tels que la gomme Konjac, la gomme de guar, l'hydroxypropylguar, l'hydroxypropylguar modifié par des groupements méthylcarboxylate de sodium, et le chlorure de guar hydroxypropyl triméthyl ammonium, et
- la polyéthylène-imine.

12. Composition selon l'une quelconque des revendications précédentes, dans laquelle les unités hydrosolubles ou hydrodispersibles ont une masse molaire allant de 1 000 g/mole à 10 000 000 g/mole lorsqu'elles constituent le squelette hydrosoluble d'un polymère greffé, ou une masse molaire allant de 500 g/mole à 500 000 g/mole lorsqu'elles constituent un bloc d'un polymère multiblocs ou lorsqu'elles constituent les greffons d'un polymère greffé.

13. Composition selon l'une quelconque des revendications précédentes, dans laquelle les unités à LCST sont constituées d'un ou plusieurs des polymères suivants :
- les polyéthers tels que le polyoxyde d'éthylène (POE), le polyoxyde de propylène (POP), et les copolymères statistiques d'oxyde d'éthylène (OE) et d'oxyde de propylène (OP),
- le polyvinylméthyléther,
- les dérivés polymériques et copolymériques N-substitués de l'acrylamide ayant une LCST ; et
- le poly N-vinylcaprolactame et les copolymères de N-vinylcaprolactame.

14. Composition selon la revendication 13, dans laquelle les unités à LCST sont constituées de copolymères statistiques d'oxyde d'éthylène (OE) et d'oxyde de propylène (OP) représentés par la formule :
(OE)ₘ(OP)ₙ
dans laquelle m est un nombre entier allant de 1 à 40, de préférence 2 à 20, et n est un nombre entier allant de 10 à 60, de préférence de 20 à 50.

15. Composition selon la revendication 14, dans laquelle la masse molaire des unités LCST est de 500 à 5300 g/mole, de préférence de 1500 à 4000 g/mole.

16. Composition selon la revendication 13, dans laquelle les unités à LCST sont des dérivés polymériques ou copolymériques N-substitués de l'acrylamide ayant une LCST tels que le poly N-isopropyl acrylamide, le poly N-éthyl acrylamide et les copolymères de N-isopropyl acrylamide ou de N-éthyl acrylamide et d'un monomère vinylique répondant à la formule (I) ou (II) donnée dans la revendication 4 ou 5, ou d'un monomère choisi parmi l'acide (méth)acrylique, l'acide vinylsulfonique, l'acide (méth)allylsulfonique, l'acide et l'anhydride maléique, l'acide vinylphosphonique, l'acide crotonique, l'acide itaconique, la (méth)acrylamide, la vinylpyridine, l'alcool vinylique, les N-vinyllactames tels que la N-vinylpyrrolidone, le styrène et ses dérivés, le chlorure de diméthyldiallyl ammonium, la N-vinylacétamide, la N-méthyl N-vinylacétamide, la N-vinylformamide, la N-méthylvinylformamide, les vinyléthers, l'acétate de vinyle, l'acrylonitrile, la caprolactone, le chlorure de vinyle, le chlorure de vinylidène.

17. Composition selon la revendication 13, dans laquelle lesdits dérivés ont une masse molaire allant de 1000 g/mole à 500 000 g/mole.

18. Composition selon la revendication 13, dans laquelle les unités à LCST sont constituées par un poly-N-vinylcaprolactame ou un copolymère de N-vinylcaprolactame et d'un monomère vinylique répondant à la formule (I) ou (II) donnée dans les revendications 4 ou 5, ou d'un monomère choisi parmi l'acide (méth)acrylique, l'acide vinylsulfonique, l'acide (méth)allylsulfonique, l'acide maléique, l'anhydride maléique, l'acide vinylphosphonique, l'acide crotonique, l'acide itaconique, la (méth)acrylamide, la vinylpyridine, l'alcool vinylique, les N-vinyllactames tels que la N-vinylpyrrolidone, le styrène et ses dérivés, le chlorure de diméthyldiallylammonium, la N-vinylacétamide, la N-méthyl-N-vinylacétamide, la N-vinylformamide, la N-méthylvinylformamide, l'acétate de vinyle, les vinyléthers, l'acrylonitrile, la caprolactone, le chlorure de vinyle, le chlorure de vinylidène.

19. Composition selon la revendication précédente, pour laquelle la masse molaire des unités à LCST est de 1000 g/mole à 500 000 g/mol.

20. Composition selon l'une quelconque des revendications 3 à 19, dans laquelle la proportion massique des unités à LCST du polymère est de 5 à 70 %, de préférence de 20 à 65 % et mieux encore de 30 à 60 % par rapport au polymère.

21. Composition selon l'une quelconque des revendications 3 à 20, dans laquelle la température de démixtion des unités à LCST est de 10 à 35°C pour une concentration massique des unités à LCST dans l'eau de 1 %.

22. Composition selon l'une des revendications précédentes, **caractérisée en ce que** le polymère à unités LCST est présent en une teneur en matières sèches allant de 0,01 % à 20% en poids de préférence de 0,1 à 15% en poids par rapport au poids total de la composition.

23. Composition selon l'une des revendications précédentes, **caractérisée en ce qu'**elle comprend un polymère filmogène additionnel.

24. Composition selon la revendication précédente, **caractérisée en ce que** le polymère filmogène additionnel est présent en une teneur en matière sèche allant de 5% à 70% en poids par rapport au poids total de la composition, mieux de 10 à 60% en poids et encore mieux de 20% à 50%.

25. Composition selon l'une des revendications précédentes, **caractérisée en ce que** le milieu aqueux représente de 5 à 95 % en poids par rapport au poids total de la composition, de préférence de 20 à 80% en poids.

26. Composition selon l'une des revendications précédentes, **caractérisée en ce qu'**elle comprend une matière colorante.

27. Composition selon la revendication précédente, **caractérisée en ce que** la matière colorante est présente en une teneur allant de 0,01 % à 10% en poids, par rapport au poids total de la composition.

28. Procédé cosmétique de maquillage ou soin non thérapeutique des ongles comprenant l'application sur les ongle d'au moins une couche d'une composition de vernis à ongles selon l'une des revendications 1 à 27.

29. Utilisation d'un polymère hydrosoluble ou hydrodispersible comprenant des unités hydrosolubles ou hydrodispersibles et des unités à LCST, lesdites unités à LCST présentant dans l'eau une température de démixtion de 5 à 40 °C à une concentration massique de 1 %, dans une composition de vernis à ongles comprenant un milieu aqueux cosmétiquement acceptable, pour conférer à ladite composition une homogénéité de couleur dans le temps, en particulier à des températures supérieures à 25°C.
